# EUROPEAN PATENT SPECIFICATION

(11) **EP 4 211 102 B1**
(45) Date of publication and mention of the grant of the patent: **17.07.2024**
(21) Application number: 21787132.6
(22) Date of filing: 09.09.2021
(51) Int. Cl.: C07C 17/04, C07C 17/25, C07C 19/01, C07C 21/04

(54) **PROCESSES FOR PREPARING PENTACHLOROPROPANE AND TETRACHLOROPROPENE FROM DICHLOROPROPENE**
VERFAHREN ZUR HERSTELLUNG VON PENTACHLOROPROPAN UND TETRACHLOROPROPEN AUS DICHLOROPROPEN
PROCEDES DE PREPARATION DU PENTACHLOROPROPANE ET DU TETRACHLOROPROPENE A PARTIR DU DICHLOROPROPENE

(30) Priority: 11.09.2020 US 202063077252 P
(43) Date of publication of application: 19.07.2023
(73) Proprietor: Blue Cube IP LLC, Clayton, Missouri 63105 (US)
(72) Inventor: TIRTOWIDJOJO, Max, Clayton, Missouri 63105 (US); MYERS, John D., Clayton, Missouri 63105 (US); SELL, Marc, Clayton, Missouri 63105 (US); SALZBAUER, Frank, Clayton, Missouri 63105 (US); DETJEN, Imke, Clayton, Missouri 63105 (US)
(74) Representative: Greaves Brewster LLP
(86) International application number: PCT/US2021/049669
(87) International publication number: WO 2022/056129

(56) References cited:
- CN-A- 101 955 414
- CN-B- 104 496 746

## Description

### CROSS REFERENCE

This application claims the benefit of U.S. provisional application number 63/077,252 filed on September 11, 2021.

### FIELD

The present disclosure generally relates to processes for preparing halogenated alkanes and alkenes, and in particular chlorinated alkanes and alkenes.

### BACKGROUND

Halogenated alkanes and alkenes are useful intermediates for producing compounds used in many products including agricultural products, pharmaceuticals, cleaning solvents, blowing agents, solvents, gums, silicones, and refrigerants.

Such halogenated alkanes and alkenes can vary in the size of the base carbon chain, and the number and placement of the halogens along the chain. One subset of highly sought halogenated alkenes are chloropropenes, in particular 1,1,2,3-tetrachloropropene and 2,3,3,3-tetrachloropropene. Pentachloropropanes produced as intermediates to these compounds may also be of value. CN 101955414 discloses a process for producing such pentachloropropanes starting from 2,3-dichloropropene.

### BRIEF DESCRIPTION OF FIGURES

In order to describe the present disclosure and its advantages and features, a more particular description of the principles briefly disclosed herein will be rendered by reference to specific embodiments which are illustrated in the appended drawings. Understanding that these drawings depict only exemplary embodiments of the disclosure and are not therefore to be considered to be limiting of its scope, the principles herein are described and explained with additional specificity and detail through the use of the accompanying drawings in which:
Figure 1 is a schematic of one exemplary embodiment of a process for preparing a mixture of 1,1,2,3-tetrachloropropene and 2,3,3,3-tetrachloropropene from 1,3-dichloropropene, comprising two chlorination reactors and two dehydrochlorination reactors; and
Figure 2 is a schematic of one exemplary embodiment of a process for preparing chloroaliphatic compounds from a crude dichloropropene feed stream comprising a single chlorination reactor and two dehydrochlorination reactors.

### DETAILED DESCRIPTION

Various embodiments of the disclosure are discussed in detail below. While the concepts of the present disclosure are susceptible to various modifications and alternative forms, specific embodiments thereof have been shown by way of example in the drawings and will be described herein in detail. It should be understood, however, that there is no intent to limit the concepts of the present disclosure to the particular forms disclosed, but on the contrary, the intention is to cover all modifications, equivalents, and alternatives consistent with the present disclosure and the appended claims.

In the drawings, some structural or method features may be shown in specific arrangements and/or orderings. However, it should be appreciated that such specific arrangements and/or orderings may not be required. Rather, in some embodiments, such features may be arranged in a different manner and/or order than shown in the illustrative figures. Additionally, the inclusion of a structural or method feature in a particular figure is not meant to imply that such feature is required in all embodiments and, in some embodiments, it may not be included or may be combined with other features.

### Introduction

Fluorocarbons are fluorinated hydrocarbons that may be used in a number of different products, such as refrigerants, and so have high demand and economic value. In order to form these fluorocarbons, chlorinated alkane and alkene compounds may be employed, and are often referred to as intermediates. These intermediate compounds themselves are valuable, and as more cost effective ways are found for their preparation the overall costs for forming the fluorocarbons are reduced. Further, producing such compounds from less costly raw materials and with more efficient processes is desired.

Particular chlorinated compounds which may be used as intermediates include, for example, 1,1,2,3-tetrachloropropene and 2,3,3,3-tetrachloropropene. Conventional processes for preparing 1,1,2,3-tetrachloropropene require extensive purifications, high manufacturing costs, and can generate large amounts of waste. Developing a process that can cost effectively and reproducibly prepare 1,1,2,3-tetrachloropropene at a reduced overall manufacturing cost may be desirable.

The particular compound 1,3-dichloropropene is a byproduct of various processes or reactions and may sometimes be considered a waste product. Disclosed herein is a process for converting 1,3-dichloropropene to at least one of 1,1,1,2,3-pentachloropropane or 1,1,2,2,3-pentachloropropane. Embodiments are also disclosed for further converting the pentachloropropanes, such as pentachloropropane isomers 1,1,1,2,3-pentachloropropane or 1,1,2,2,3-pentachloropropane, to at least one of 1,1,2,3-tetrachloropropene or 2,3,3,3-tetrachloropropene. The process may include one or two successive alternating chlorination reactors alternated with one or two successive dehydrochlorination reactors.

In particular, the process may begin with a first chlorination reaction where 1,3-dichloropropene is reacted with a chlorination agent to produce a chlorination reaction product comprising 1,1,2,3-tetrachloropropane. This chlorination reaction product is then reacted with a dehydrochlorination reagent or catalyst in a first dehydrochlorination reaction to produce a dehydrochlorination reaction product comprising trichloropropenes, such as 1,1,3-trichloropropene.

The dehydrochlorination reaction product comprising trichloropropenes can then be subjected to a second chlorination reaction by reacting with a chlorination agent to form a second chlorination reaction product including pentachloropropane, such as 1,1,1,2,3-pentachloropropane, 1,1,2,2,3-pentachloropropane, and mixtures thereof. Thereafter, in a second dehydrochlorination reaction, this second chlorination reaction product may be reacted with a dehydrochlorination reagent or catalyst to form 1,1,2,3-tetrachloropropene, 2,3,3,3-tetrachloropropene, or mixtures thereof.

Various other products and may be formed in the above described process, which may be separated, recycled or subjected to other reactions.

The aforementioned reactions may be carried out with pure, or impure 1,3-dichloropropene. In industry, the 1,3-dichloropropene may often be produced in a crude stream, referred to herein as a crude 1,3-dichloropropene stream. This crude stream may include other chloroaliphatic compounds, such as 1,2-dichloropropane, and dichloropropene isomers other than 1,3-dichloropropene, such as 3,3-dichloropropene or 2,3-dichloropropene. These other compounds in the crude stream may undergo other reactions. For instance, the 2,3-dichloropropene in a chlorination reaction produces 1,2,2,3-tetrachloropropane. The dehydrochlorination of 1,2,2,3-tetrachloropropane may be carried out in the presence of a phase transfer catalyst to facilitate the reaction. Alternatively, the 1,2,2,3-tetrachloropropane may be purged from the process. Further alternatively, the 1,3-dichloropropene may be removed or purified from the crude dichloropropene stream, and other compounds and other isomers removed. Alternatively, the 2,3-dichloropropene may also be removed from the crude stream to avoid the later reactions involving 1,2,2,3-tetrachloropropane.

### Chlorination of 1,3-dichloropropene

The processes commence by reacting 1,3-dichloropropene with a chlorinating agent in a first chlorination reaction. This may be carried out by preparing 1,3-dichloropropene in a liquid phase, where the 1,3-dichloropropene may be dissolved in a solvent. The chlorinating agent may be in the gas or liquid phase. The gas-phase chlorinating agent is absorbed into the liquid phase where the chlorination reaction occurs.

### 1,3-dichloropropene

The 1,3-dichloropropene may be provided in a pure or in an impure feed. The pure feed may have 1,3-dichloropropene from at least about 90 mol%, alternatively at least about 95 mol%, alternatively at least about 98 mol%, alternatively at least about 99 mol%, alternatively about 100 mol%. The 1,3-dichloropropene may also be a mixture of cis- and trans- isomers, or alternatively only cis- or only trans- isomers.

In the impure feed, the 1,3-dichloropropene may include other compounds, such as other chloroaliphatic compounds, such as chloroalkene or chloralkane compounds, and isomers of dichloropropene other than 1,3-dichloropropene. Such chloroaliphatic compounds may include a base hydrocarbon chain such as an alkane or alkene, which may include 2 to 5 carbons, alternatively from 2 to 4 carbons, or alternatively 2-3 carbons, or alternatively 2, 3, 4 or 5 carbons, and may be a ethane, ethene, propane, propene, butane, butene, pentane, and/or pentene, and mixtures thereof. The base hydrocarbon chain may be functionalized with one or more chlorines, and/or additional halogens such as fluorine. For instance, the base hydrocarbon chain may be functionalized with 1, 2, 3, 4, 5, or 6 chlorines. These may be provided at any of the positions along the hydrocarbon chain.

In the impure feed, or crude stream, the 1,3-dichloropropene may include other compounds. Such crude 1,3-dichloropropene may be a product or byproduct of another reaction. The crude 1,3-dichloropropene stream may include both cis-1,3-dichloropropene and trans-1,3-dichloropropene, other chloroaliphatic compounds and other isomers of dichloropropene. Other chloroaliphatic compounds may include for instance 3-chloropropene, 1,2-dichloropropane, 1,2,3-trichloropropane, or isomers of dichloropropene other than 1,3-dichloropropene, which may include for example one or more of 3,3-dichloropropene or 2,3-dichloropropene. In some embodiments, the 2,3-dichloropropene may be separated out from the feed to avoid the production of 1,2,2,3-tetrachloropropane.

The crude 1,3-dichloropropene may include 1,3-dichloropropene from about 25 mol% to about 70 mol%, alternatively from about 35 mol% to about 55 mol%, which may be entirely cis-, entirely trans-, or about equal amounts of each. The crude 1,3-dichloropropene may include 2,3-dichloropropene from about 1 mol% to about 20 mol%, alternatively from about 1 to about 10 mol%, alternatively from about 5 to about 10 mol%, alternatively from about 2 mol% to about 5 mol%, and may include 1,2-dichloropropane up to about 60 mol%, alternatively up to about 50 mol%, alternatively up to about 40 mol%, from about 40 mol% to about 60 mol%, alternatively from about 45 mol% to about 55 mol%, or may contain 0 mol% when not present.

In carrying out the reaction, the 1,3-dichloropropene may be added portionwise to a chlorination reactor. In another embodiment, the 1,3-dichloropropene is added continuously to the chlorination reactor.

The 1,3-dichloropropene is typically dry or anhydrous, or may be carried out with a small amount of water. Accordingly, the feed may include less than about 1000 ppm water, less than about 100 ppm water, or less than 10 ppm water. The 1,3-dichloropropene may be dried with a molecular sieve, silica gel, alumina, and mixtures of the same. Alternatively, the 1,3-dichloropropene may be dried by azeotropic distillation.

A solvent or a mixture of solvents may be employed in the chlorination reaction to dissolve the 1,3-chloropropene. Non-limiting examples of suitable solvents include carbon tetrachloride (CCl₄), 1,2-dichloropropane, or mixtures thereof.

### Chlorinating agent

A wide variety of chlorinating agents may be used in the above mentioned chlorination reaction, as well as in any chlorination reactions disclosed herein. The chlorinating agent reacts with the alkenes that are present including chloroalkenes. The chlorinating agent may also react with the alkanes that may be present including chloroalkanes. Useful chlorinating agents include chlorine, sulfuryl chloride (SO₂Cl₂), or a combination thereof. When chlorine is used, the total reactor pressure is at or greater than atmospheric pressure. The chlorinating agent may be a gas, a liquid or a combination thereof. The chlorine gas may be bubbled through the liquid phase reactant and/or solvent, or pre-dissolved in the liquid-phase reactant or solvent prior to introduction into the reactor or pre-dissolved in a recycle stream.

The chlorinating agent is typically provided in excess relative to 1,3-chloropropene and/or other chloroaliphatic reactants, but may also be used in substoichiometric amounts. Generally, the mole ratio of the chlorinating agent to the 1,3-dichloropropene ranges from about 0.9:1 to about 10:1, alternatively from about 1:1 to about 5:1, from about 1.05:1 to about 3:1, from about 1.1:1 to about 2:1, or from about 1.2:1 to about 1.5:1. The chlorine gas may be bubbled through the liquid phase comprising the 1,3-chloropropene.

### Chlorination Reaction

The chlorination may be run in a batch mode, semi-batch mode, or a continuous mode, with continuous mode being a particular embodiment. The reaction may be carried out in a reactor made of carbon steel or an inert material, such as hastelloy, tantalum, or a glass lined reactor. A continuous mode reactor may include a continuous stirred tank reactor, plug flow reactor, or a jet loop reactor.

In order to increase the efficiency of the process, the contents of the reactor may be stirred. Non-limiting methods for stirring the liquid phase reaction mixture include jet stirring, mechanical impellers, packed columns, baffles, or combinations thereof. Non-limiting examples of methods to mix the contents of the reactor and provide increased gas absorption into the liquid phase reaction mixture include jet stirring using at least one eductor, jet stirring comprising at least one nozzle and at least one eductor, jet stirring wherein jet stirring comprises at least one nozzle, reactors with specially designed baffles, and combinations thereof.

Jet mixing utilizing at least one nozzle withdraws a portion of the liquid phase of the liquid phase reaction mixture from the reactor and pumps the liquid phase reaction mixture back into the reactor through at least one nozzle. This creates turbulence in the liquid phase reaction mixture and increases mixing. The at least one nozzle may be positioned below the surface of the liquid phase reaction mixture, at the surface of the liquid phase reaction mixture or directed through the gas phase into the liquid phase reaction mixture.

In other embodiments, a draft tube may be utilized in the process. The draft tube provides an internal circulation of the liquid phase reaction mixture within the reactor. The circulation may be induced by energy from the at least one liquid jets, from the at least one gas educting nozzles, from rising gas bubbles within the reactor, or a combination thereof.

The reaction may utilize a device that sonicates the reaction mixture. Sonication uses sound energy to agitate particles and liquids in the process and increases the kinetics of the process.

The chlorination reaction may be conducted to maintain the temperature from about 0°C to about 110°C, alternatively from about 20°C to about 80°C, alternatively from about 30°C to about 60°C using an internal or external heat exchanger.

The chlorination reaction may be carried out at pressures from about 0 to about 6,90 MPa (0-1000 psig), alternatively from about 0 to about 3,45 MPa (0-500 psig) or from about 0 to about 1,38 MPa (0-200 psig).

The chlorination reaction process is allowed to proceed until the reaction is sufficiently complete. In order to determine the completeness, chromatography (e.g., gas chromatography) may be used to monitor the progress of the reaction. The duration of the reaction may range from about 1 minute to about 24 hours. In some embodiments, the duration of the reaction may range from about 10 minutes to about 12 hours, alternatively from about 15 minutes to about 3 hours, alternatively from about 30 minutes to about 2 hours, alternatively from about 20 minutes to about 1 hours.

### Chlorination Reaction Product

The 1,3-dichloropropene reacts with the chlorinating agent to generate a chlorination reaction product having chloroalkanes, including tetrachloroalkanes. Such tetrachloroalkanes include 1,1,2,3-tetrachloropropane.

In other embodiments, at least 50 mol%, or at least 70%, or at least 90%, or at least 95%, or at least 98% of the 1,3-dichloropropene that enters the chlorination reactor is converted into 1,1,2,3-tetrachloropropane. Yield may be defined as the moles of product divided by the moles of reactant multiplied by 100%. In the present case, the yield is the moles of 1,1,2,3-tetrachloropropane produced divided by the moles of 1,3-dichloropropene reacted multiplied by 100%, and such yield, may be at least 50%, at least 80%, at least 90%, or at least 95%.

The 1,1,2,3-tetrachloropropane in the chlorination reaction product may be separated or purified. The 1,1,2,3-tetrachloropropane may be purified to at least 99%, or alternatively at least 99.9%. Any unreacted 1,3-dichloropropene, unreacted chlorine or both unreacted 1,3-dichloropropene and unreacted chlorine may be separated out and at least partially or fully recycled back to the chlorination reactor or feed. A purification step may include providing the chlorination reaction product to a separator where two exit streams are formed, one having 1,1,2,3-tetrachloropropane and the other 1,3-dichloropropene. Further separations may be conducted which remove compounds or fractions lighter or heavier than 1,1,2,3-tetrachloropropane. Heavier compounds are those having a higher boiling point and generally a higher molecular weight, whereas lighter compounds have a lower boiling point and generally have a lower molecular weight as compared to 1,1,2,3-tetrachloropropane. The lighter fraction may be recycled to the reactor. To the extent HCl is produced or present in the reaction product, it may be separated out. For example, the light fraction may include anhydrous HCl. The anhydrous HCl can be and often is separated from the light fraction, before a light fraction is recycled to the chlorination reactor. Alternatively, a portion of the light fraction may be purged from the process to minimize HCl accumulation.

The separator may be a distillation column, a multistage distillation column, and/or an evaporator. The separator may include a reboiler and/or a bottom stage. In some embodiments, a side draw column or a distillation column which provides an outlet stream from an intermediate stage or a dividing wall column (DWC), which is a single shell, fully thermally coupled distillation column capable of separating mixtures of three or more components into high purity products, i.e., product streams, may be used as a separator.

### Additional Crude Stream Reactions

When the 1,3-dichloropropene is provided as part of an impure stream, such as a crude 1,3-dichloropropene stream, the other compounds in the stream may undergo reactions to produce other compounds in the chlorination reaction product. As mentioned, the crude stream may include other chloroaliphatic compounds such as 1,2-dichloropropane and/or isomers of dichloropropene including 3,3-dichloropropene, 2,3-dichloropropene, and mixtures thereof.

The chlorination of 2,3-dichloropropene produces 1,2,2,3-tetrachloropropane as a product. The 1,2,2,3-tetrachloropropane may also be used as a solvent for the chlorination reaction. Additionally, 1,2-dichloropropane can partially chlorinate to 1,2,3-trichloropropane in the chlorination reaction. These compounds may be separated out from the chlorination reaction product using separators as described previously or may undergo further reactions in the dehydrochlorination reaction. For instance, a phase transfer catalyst may be employed for the dehydrochlorination of 1,2,2,3-tetrachloropropane. Alternatively, the separated 1,2,2,3-tetrachloropropane may be recycled to the first chlorination reactor, where it may be partially chlorinated, or fed to a chlorination reactor wherein free radical chlorination is carried out employing a free radical initiator such as Azobisisobutyronitrile ("AIBN"). Alternatively the separated 1,2,2,3-tetrachloropropane may be provided to a separate dehydrochlorination reactor for dehydrochlorination which may include the presence of a phase transfer catalyst. When 1,2,2,3-tetrachloropropane is chlorinated useful 1,1,2,2,3-pentachloropropane is formed. Additionally 1,2,3-trichloropropane dehydrochlorinates to 2,3-dichloropropene, which can be recycled back to the first chlorination reaction.

### Dehydrochlorination of the Chlorination Reaction Product

As mentioned above, as a result of the chlorination reaction of 1,3-dichloropropene, a chlorination reaction product comprising 1,1,2,3-tetrachloropropane is produced. The chlorination reaction product may be subject to a first dehydrochlorination reaction by reacting it with a dehychlorination reagent.

### Dehydrochlorination reagent

The dehydrochlorination reagent may include a base, such as an inorganic base which may be in aqueous form. The base may be produced by a chloroalkali process and may include dissolved salts in addition to the basic constituents.

The inorganic base may be an alkali metal or alkali earth metal base. Non-limiting examples of these alkali metal or alkali earth metal bases may include LiOH, NaOH, KOH, Ba(OH)₂, Ca(OH)₂, Na₂CO₃, K₂CO₃, NaHCO₃, KHCO₃, or combinations thereof. In particular, the alkali or alkali earth metal base may include NaOH, KOH, or combinations thereof, and in particular NaOH. During the dehydrochlorination reaction the base may react with one or more of the chlorines or other halogens of the compounds in the chlorination reaction product and may thereby form an alkali or alkali earth metal chloride salt. A particular salt that may be formed as a result of the dehydrochlorination reaction described herein is sodium chloride.

Generally, the dehydrochlorination reagent is aqueous, and the concentration of the dehydrochlorination reagent in the reagent solvent, such as water, may range from 5 wt% to about 50 wt%. In various embodiments, the concentration of the dehydrochlorination reagent may range from 5 wt% to about 50 wt%, from 7 wt% to about 40 wt%, from 9 wt% to about 30 wt%, or from 10 wt% to about 20 wt%. In a particular embodiment, the concentration of the dehydrochlorination reagent may range from 5 wt% to about 12 wt%.

In general, the mole ratio of the dehydrochlorination reagent to the 1,1,2,3-tetrachloropropane may range from 0.1:1.0 to about 2.0:1.0. The dehydrochlorination reagent may be provided in excess of the 1,1,2,3-tetrachloropropane. The dehydrochlorination reagent to 1,1,2,3-tetrachloropropane ratio may be about equal but with excess reagent. In various embodiments, the mole ratio of the dehydrochlorination reagent to the 1,1,2,3-tetrachloropropane may range from 0.1:1.0 to about 2.0:1.0, from 0.5:1.0 to about 1.5:1.0, or from 0.9:1.0 to about 1.1:1.0. In a particular embodiment, the mole ratio of the dehydrochlorination reagent to the 1,1,2,3-tetrachloropropane may be about 1.05:1.0. When other components in the feed to the dehydrochlorination reactor are dehydrochlorinated, these ranges may also apply to the ratio of dehydrochlorination reagent to the total of the components that can be dehydrochlorinated.

The dehydrochlorination reagent may be a reactant and consumed in the reaction. In other embodiments the dehydrochlorination of 1,3-dichloropropene and other components in the chlorination reaction product involves a component that assists the reaction while not being consumed in the reaction, for instance, a catalyst.

Alternative to the dehydrochlorination reagent, a dehydrochlorination catalyst may be employed to carry out the dehydrochlorination reaction. The dehydrochlorination catalyst may be a Lewis acid catalyst. The Lewis acid catalyst may be dissolved in a solvent prior to being added to the reactor. At least part of the at least one Lewis acid catalyst may be in homogeneous or heterogeneous form. In various embodiments, the Lewis acid catalyst comprises gallium, iron, or combinations thereof. Non-limiting examples of these Lewis acid catalysts may be gallium metal, a gallium salt, a gallium alloy, iron metal, an iron salt, an iron alloy, or combinations of two or more thereof. Non-limiting examples of the forms or configuration of Lewis acid catalyst may be a dissolved species in the liquid phase, a species deposited on a solid support, a packing, an unstructured packing, a foil, a sheet, a screen, a wool, a wire, a ball, a plate, a pipe, a rod, a bar, a salt, or a powder. The weight% (wt%) of the Lewis acid catalyst in the reaction mixture may range from about 0.0001 wt% to about 2.0 wt%.

### Dehydrochlorination Reaction

The dehydrochlorination process may be run in a batch mode or a continuous mode. In another embodiment, the process in continuous modes may be stirred by the methods disclosed herein to improve the mixing of the biphasic system. One particular method for ensuring the contents of the reactor are adequately mixed may be utilizing a jet stirred reactor which mixes the contents of the reactor without an impeller, i.e., jet mixing. The jet mixing is caused by feeding fresh liquid feed, product effluent stream, a recycle stream or combinations thereof to at least one nozzle. In this jet stirred reactor system, the liquid materials comprising internal recycle, fresh feed or both are introduced vertically, tangentially or radially into the reactor by means of an external pump.

The temperature of the process may vary depending on concentration of the compounds involved, the type of chosen base, and the concentration of the base. Generally, the temperature of the process may be generally from about 20°C to about 120°C, alternatively from about 50°C to about 110°C, or from about 60°C to about 100°C.

Generally, the pressure may range from about 0 to about 6,90 MPa (0-1000 psig), alternatively from about 0 to about 3,45 MPa (0-500 psig) or from about 0 to about 1,38 MPa (0-200 psig).

The process may be conducted under an inert atmosphere such as nitrogen, argon, or helium.

The reaction may be allowed to proceed for a sufficient period of time until the reaction is complete.

### Dehydrochlorination Reaction Product

The dehydrochorination reaction product form the dehydrochlorination reaction includes trichloropropenes. These trichloropropenes include 1,1,3-trichloropropene, 1,2,3-trichloropropene (cis and/or trans), 2,3,3-trichloropropene, 1,3,3-trichloropropene and mixtures thereof. The conversion of 1,1,2,3-tetrachloropropane may be at least 85% or at least 90% or at least 95% or at least 98%. The selectivity to trichloropropenes may be at least 85% or at least 90% or at least 95% or at least 98%. The relative amounts of the trichloropropene mixture may depend on the process by which they are produced. For instance, the 1,1,3-trichloropropene may range from about 10 to about 90 mol%, about 40 to about 70 mol%, alternatively from about 45 to about 65 mol%, alternatively from about 50 to about 60 mol% of the reaction product, the 2,3,3-trichloropropene may range from about 10 to about 50 mol%, from about 15 to about 25 mol%, alternatively about 17 to about 22 mol% of the reaction product, and the 1,2,3-trichloropropene may range from about 5 to about 50 mol%, from about 10 to about 35 mol%, or alternatively from about 10 to about 20mol%, of either cis and/or trans, or alternatively with approximately equal amounts of cis and trans forms (5-10% trans, and 5-10% cis for example), with the remainder being other trichloropropene isomers or other chloroaliphatics.

The dehydrochorination reaction product may be purified to increase the concentration of trichloropropenes. The product may be purified or otherwise concentrated to at least 85 mol%, alternatively at least 87 mol%, alternatively at least 89 mol%, alternatively at least 95 mol%, alternatively at least 99 mol%, alternatively about 100 mol%. The reaction product may be purified or separations carried out such that all trichloropropenes may be provided to the next chlorination reaction.

Separating the trichloropropenes from the reactor may include at least two or three product streams. In various embodiments, separating the trichloropropenes may produce four, five, or more product streams depending on the separation device utilized. Separators as described herein may be employed.

A portion of various product streams after separation are optionally recycled back into the dehydrochlorination reactor to provide increased kinetics, increased efficiencies, and reduced overall cost of the process. In an embodiment, any unreacted or newly formed 1,1,2,3-tetrachloropropane may be recycled back to a dehydrochlorination reactor.

### Additional Reaction Products from Crude Dichloropropene

In embodiments where the feed material to the first chlorination reaction also includes 2,3-dichloropropene, such as where the feed is crude dichloropropene, the chlorination of 2,3-dichloropropene produces 1,2,2,3-tetrachloropropane in the first chlorination step. The 1,2,2,3-tetrachloropropane may also be formed in the first chlorination step if the feed material also contains 1,2-dichloropropane, as it may be in the crude dichloropropene. The 1,2-dichloropropane can partially chlorinate to 1,2,3-trichloropropane, which in turn, dehydrochlorinates to 2,3-dichloropropene in the first dehydrochlorination step. This formation of 2,3-dichloropropene in the first dehydrochlorination step can then be recycled to the first chlorination step.

Accordingly, when the first chlorination reaction includes 2,3-dichloropropene such as in crude dichloropropene, as mentioned above, the chlorination product may contain 1,2,2,3-tetrachloropropane in the chlorination reaction as well as 1,2,3-trichloropropane. The 1,2,2,3-tetrachloropropane may be fed to the dehydrochlorination reaction, it may be separated into a separate stream and subject to a dehydrochlorination reaction separately in a separate dehydrochlorination reactor or it may be removed from the process completely as a byproduct. When 1,2,2,3-tetrachloropropane is present in the chlorination reaction product, a phase transfer catalyst may be helpful or necessary for dehydochlorination of the 1,2,2,3-tetrachloropropane. A phase transfer catalyst may be used to improve the kinetics of 1,2,2,3-tetrachloropropane dehydrochlorination, either in the dehydrochlorination reaction described above or in a separate dehydrochlorination reaction. Dehydrochlorination of 1,2,2,3-tetrachloropropane produces 1,2,3-trichloropropene isomers, which are useful compounds in subsequent reaction steps. Alternatively, 1,2,2,3-tetrachloropropane may be fed to the dehydrochlorination reaction without phase transfer catalyst, in which case it will be essentially inert.

The 1,2,2,3-tetrachloropropane leaving the dehydrochlorination reactor may be separated from the dehydrochlorination product, or it may be fed with the trichloropropenes leaving the dehydrochlorination reaction to a chlorination reaction. If fed to a chlorination reactor, 1,2,2,3-tetrachloropropane may act as a solvent and/or may be at least partially chlorinated to 1, 1,2,2,3-pentachloropropane. If 1,2,2,3-tetrachloropropane is separated from the dehydrochlorination product, it may be sent to a separate chlorination reaction (in a separate reactor) in which a free radical initiator such as AIBN is added to improve free radical chlorination kinetics. Alternatively, a small amount of phase transfer catalyst could be added to the first dehydrochlorination step to convert at least a portion of the 1,2,2,3-tetrachloropropane such that its accumulation is limited to a tolerable level. In some embodiments, the 1,2,2,3-tetrachloropropane may be simply purged from the process in a purge stream that is selected to minimize the loss of other molecules which may be considered more valuable. The purge stream may be created by a distillation step or other separator.

Accordingly, in some instances the dehydrochlorination may utilize a phase transfer catalyst. Non-limiting examples of phase transfer catalysts may be quaternary ammonium salts, phosphonium salts, and pyridinium salts. In some embodiments, the phase transfer catalyst may be a quaternary ammonium salt. Non-limiting examples of suitable salts are chlorides, bromides, iodides, or acetates. Non-limiting examples of quaternary ammonium salts include trioctylmethylammonium chloride (Aliquat^{®} 336), trioctylmethylammonium bromide, dioctyldimethylammonium chloride, dioctyldimethylammonium bromide, Arquad 2HT-75, benzyldimethyldecylammonium chloride, benzyldimethyldecylammonium bromide, benzyldimethyldecylammonium iodide, benzyldimethyltetradecylammonium chloride, dimethyldioctadecylammonium chloride, dodecyltrimethylammonium chloride, tetrabutylammonium chloride, tetrabutylammonium bromide, tetrabutylammonium iodide, tetrabutylammonium acetate, tetrahexylammonium chloride, tetraoctylammonium chloride, tridodecylmethylammonium chloride, tetraethylammonium chloride, tetraethylammonium bromide, tetraethylammonium iodide, or combinations thereof. In some embodiments, more than one phase transfer catalyst is used. In a preferred embodiment, the phase transfer catalyst is trioctylmethylammonium chloride (Aliquat^{®} 336).

The amount of the phase transfer catalyst may range from 0.001 wt% to about 10.0 wt% based on the total weight of the components, alternatively from 0.05 wt% to 7.5 wt%, from 0.02 wt% to about 2.5 wt%, or from 0.01 wt% to about 1.0 wt%.

When 1,2,3-trichloropropane is produced from the chlorination reaction product, such as when 1,2-dichloropropane is in the crude dichloropropene and partially chlorinates to 1,2,3-trichloropropane, or when 3-chloropropene is in the crude dichloropropene and chlorinates to 1,2,3-trichloropropane, the 1,2,3-trichloropropane can react with the dehydrochlorination reagent. In such a dehydrochlorination reaction, the 1,2,3-trichloropropane converts to 2,3-dichloropropene, which may then be recycled back to the chlorination reactor.

While the reaction and/or purge or recycle of 1,2,2,3-tetrachloropropane may be carried out as discussed herein, in many cases, the 2,3-dichloropropene and the 1,2-dichloropropane may be removed from the crude dichloropropene in the feedstream prior to conducting any chlorination.

### Second Chlorination Process

Subsequent the dehydrochlorination reaction, a second chlorination reaction may be carried out. This may be carried out in a different or second chlorination reactor, different from the reactor in which the first chlorination reaction is carried out. Alternatively, the first and second chlorination reactions can be carried out in a single, or same, chlorination reactor.

In the second chlorination, the purified or unpurified reaction product from the dehydrochlorination reaction comprising the trichloropropenes may be reacted with a chlorination agent. The same or different chlorination agent may be used as in the first chlorination of 1,3-dichloropropene, and with the reactor conditions as described above, and optionally the solvents for the chlorination reaction as previously described.

The chlorination of the reaction product including the trichloropropenes results in the conversion of these trichloropropenes to a chlorination reaction product having pentachloropropanes such as 1,1,1,2,3-pentachloropropane, 1,1,2,2,3-pentachloropropane, and mixtures thereof.

The conversion of trichloropropenes and/or of 1,1,3-trichloropropene may be at least 85%, or at least 90%, or at least 95%, or at least 97%, or at least 98%. The selectivity to 1,1,1,2,3-pentachloropropane and/or 1,1,2,2,3-pentachloropropane may be at least 85% or at least 90% or at least 95% or at least 98%. The 1,1,1,2,3-pentachloropropane and/or 1,1,2,2,3-pentachloropropane in the reaction product may range from about 75 mol% to about 99 mol%, alternatively from about 80 mol% to 90 mol%, or alternatively at least 95%, alternatively at least 98%, alternatively at least 99%.

The pentachloropropane containing reaction product may be purified to increase 1,1,1,2,3-pentachloropropane and/or 1,1,2,2,3-pentachloropropane in the reaction product. The products may be separated by distillation, thus separating components with lower boiling points and/or higher boiling points than the 1,1,1,2,3-pentachloropropane and/or 1,1,2,2,3-pentachloropropane.

In some embodiments, the second chlorination reaction can be conducted simultaneously with the first chlorination reaction in the same reactor. In this case, the reaction product from the combined chlorination reactor comprises both tetrachloropropane and pentachloropropane products, including 1,1,2,3-tetrachloropropane, 1,2,2,3-tetrachloropropane 1,1,1,2,3-pentachloropropane, 1,1,2,2,3-pentachloropropane and mixtures thereof. Separation of the reaction product can be conducted using methods disclosed above, such as distillation, to produce a stream enriched in tetrachloropropanes that can be directed back to the first dehydrochlorination reaction, and a stream enriched in pentachloropropanes that can be directed to the second dehydrochlorination reaction, described below. Other product streams from such a separation may also be produced, such as light or heavy by-products.

### Second Dehydrochlorination Process

The reaction product of the second chlorination reaction including the pentachloropropanes, whether purified or unpurified, can then be subjected to a second dehydrochlorination reaction. In this second dehydrochlorination reaction, the reaction product of the second chlorination reaction is reacted with a dehydrochlorination reagent or dehydrochlorination catalyst. The dehydrochlorination reagent or dehydrochlorination catalyst can be used as described above with respect to the first dechydrochlorination, and may be the same or different from the dehydrochlorination reagent or dehydrochlorination catalyst used in the first dehydrochlorination reaction, and in the same or different dehydrochlorination reactor.

The pentachloropropane-containing chlorination reaction product having 1,1,1,2,3-pentachloropropane and/or 1,1,2,2,3-pentachloropropane produces a second dehydrochlorination product having tetrachloropropenes, including 1,1,2,3-tetrachloropropene and/or 2,3,3,3-tetrachloropropene. The 1,1,2,3-tetrachloropropene and/or 2,3,3,3-tetrachloropropene in the reaction product may range from about 75 mol% to about 99 mol%, alternatively from about 85 mol% to 95 mol%.

The conversion of 1,1,1,2,3-pentachloropropane and/or 1,1,2,2,3-pentachloropropane may be at least 85%, or at least 90%, or at least 95%, or at least 97%, or at least 98%, or at least 99%. The selectivity to 1,1,2,3-tetrachloropropene and/or 2,3,3,3-tetrachloropropene may be at least 85% or at least 90% or at least 95% or at least 98%.

The reaction product can be further purified to increase the concentration of the tetrachloropropenes or to remove unwanted components employing separators as disclosed herein. Any unreacted feed components may be recycled back to the dehydrochlorination reactor for the second dehydrochlorination reaction. The 2,3,3,3-tetrachloropropene may also be converted to 1,1,2,3-tetrachloropropene such as via an isomerization reaction and such reaction may be carried out in the presence of a catalyst.

### Exemplary Process

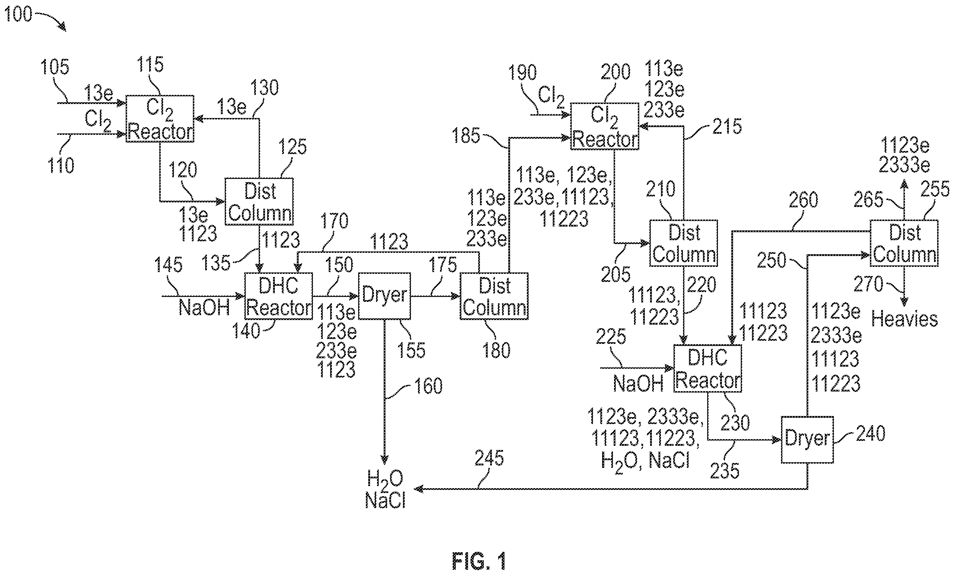

FIG. 1 illustrates an exemplary process 100 for producing at least one of 1,1,2,3-tetrachloropropene or 2,3,3,3-tetrachloropropene from 1,3-dichloropropene. As shown, an initial feed 105 comprising 1,3-dichloropropene along with a chlorination agent 110, in this case a chlorine gas, are fed to a first chlorination reactor 115 where a first chlorination reaction is carried out. While feed 105 may be considered a pure feed of 1,3-dichloroprene (and may have , in other embodiments the feed may be a crude dichloroprene having other compounds.

The first chlorination reaction product 120 comprising tetrachloroalkanes is produced, with such tetrachloroalkanes as 1,1,2,3-tetrachloropropane. This may be fed to a separator 125, which in this case is a distillation column, and where any unreacted 1,3-dichloropropene may be separated as a light fraction 130 and recycled back to the reactor 115. The purified reaction product 135 comprising 1,1,2,3-tetrachloropropane may then be fed to a first dehydrochlorination reactor 140 for a first dehydrochlorination reaction. A dehydrochlorination reagent 145, in this case aqueous NaOH, is provided to reactor 140 to react with the purified reaction product 135.

A first dehydrochlorination product 150 is produced comprising trichloropropenes. Such trichloropropenes include a mixture of 1,1,3-trichloropropene, 1,2,3-trichloropropene (cis and trans) and 2,3,3-trichloropropene. This first dehydrochlorination product 150 may be fed to a dryer 155, which may be a decanter, where any water and salts 160 such as NaCl produced or otherwise present in the reaction may be separated. In other embodiments, the first dehydrochlorination product 150 may be further dried by distillation, molecular sieve, silica or alumina. The dried first dehydrochlorination product 175 may then be provided to a separator 180, in this case a distillation column, where any unreacted 1,1,2,3-tetrachloropropane may be separated in a recycle stream 170, which may be fed back to the first dehydrochlorination reactor 140. In some embodiments the separator 125 may be omitted, in which case any residual 1,3-dichloropropene leaving the first chlorination reactor 115 may be fed through the first dehydrochlorination reactor 140 and recycled to the first chlorination reactor 115 from separator 180.

The purified dehydrochlorination product 185 may be fed to a second chlorination reactor 200 for a second chlorination reaction. A chlorination reagent 190, in this case chlorine, can be fed to the second chlorination reactor 200. A second chlorination product 205 is produced which includes pentachloropropanes such as 1,1,1,2,3-pentachloropropane and 1,1,2,2,3-pentachloropropane. This may be provided to a separator 210 which may separate out any unreacted trichloropropenes in the purified dehydrochlorination product 185 and provided via recycle stream 215 back to the second chlorination reactor 200. The purified second chlorination product 220 may be fed to a second dehydrochlorination reactor 230 along with a dehydrochlorination reagent 225, in this case aqueous NaOH for a second dehydrochlorination reaction.

The second deyhydrochlorination product 235 includes the desired intermediate tetrachloropropenes, including 1,1,2,3-tetrachloropropene and 2,3,3,3-tetrachloropropene. The second deyhydrochlorination product 235 is fed to a dryer 240 where any water and salts 245 such as NaCl produced or otherwise present from the reaction may be separated. The dried second dehydrochlorination product 250 may then be fed to a separator 255, in this case a distillation column, to produce a purified stream 265 having a higher concentration of 1,1,2,3-tetrachloropropene and 2,3,3,3-tetrachloropropene, and a stream of heavier components 270. Any unreacted pentachloropropanes in stream 270 such as 1,1,1,2,3-pentachloropropane and 1,1,2,2,3-pentachloropropane may be fed back to the dyhydrochlorination reactor 230 via recycle 260. In FIG. 1, although dryers and separators are shown for illustrative purposes, such processes may be omitted. Similarly, recycle streams may be omitted as well. Although separators 125, 180, and 210 are shown, as well as dryers 155 and 240 in the process 100, in some embodiments these may be omitted if purification or separation is not required or desired.

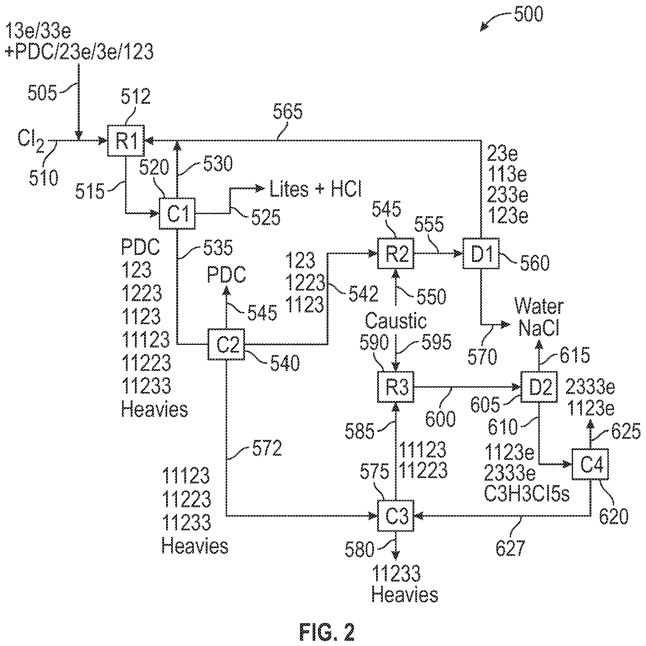

Further, the configuration of FIG. 1 may be readily converted to a configuration similar to FIG. 2 (described below), wherein a single chlorination reactor is employed for chlorination of dichloropropenes and trichloropropenes together without a second separate chlorination reactor.

FIG. 2 illustrates an exemplary process 500 for producing pentachloropropanes and/or at least one of 1,1,2,3-tetrachloropropene or 2,3,3,3-tetrachloropropene from a crude dichloropropene stream. In this process two dehydrochlorination reactions can be carried out, one directed toward reaction of trichloropropanes and tetrachloropropanes to produce dichloropropenes and trichloropropenes, respectively, and the other directed toward the reaction of pentachloropropanes to products 1,1,2,3-tetrachloropropene and/or 2,3,3,3-tetrachloropropene.

As shown, the crude dichloropropene feed stream 505 is combined with a chlorinating agent 510, in this case a chlorine gas, which is fed to a single chlorination reactor 512 where a chlorination reaction is carried out. While feed stream 505 may be crude dichloropropene stream, in other embodiments, it may be a more pure dichloropropene stream with fewer components other than 1,3-dichloropropene. Crude dichloropropene and chlorinating agent may alternatively be fed independently to reactor 512. The crude dichloropropene feed stream 505 includes 1,3-dichloropropene but also multiple other chloroaliphatic components including 1,2-dichloropropane, 3-chloropropene, 1,2,3-trichloropropane as well as isomers of dichloropropene other than 1,3-dichloropropene, including 3,3-dichloropropene and 2,3-dichloropropene.

In the first chlorination reactor 512 the 1,3-chloropropene and 3,3-chloropropene chlorinate to 1,1,2,3-tetrachloropropane. The 2,3-dichloropropene chlorinates to 1,2,2,3-tetrachloropropane, and the 1,2-dichloropropane partially chlorinates to 1,2,3-trichloropropane. The reaction product 515 may be fed to a separator 520, in this case a distillation column, to remove any light fractions 525 including HCl. Any unreacted dichloropropenes may be recycled back via recycle 530. The purified reaction product 535 includes 1,2-dichloropropane, 1,2,3-trichloropropane, 1,2,2,3-tetrachloropropane, 1,1,2,3-tetrachloropropane, 1,1,1,2,3-pentachloropropane, 1,1,2,2,3-pentachloropropane, and 1,1,2,3,3-pentachloropropane, as well as other heavy fraction components. The pentachloropropane components leaving reactor 520 are chlorination products of trichloropropenes recycled to reactor 520 from reactor 545 and the subsequent dryer 560, to be discussed below.

The purified reaction product 535 is then fed into a second separator 540, where a light stream 545 including 1,2-dichloropropane is removed and may be directed back to the chlorination reactor 512 or disposed of. A fraction 542 including 1,2,3-trichloropropane, 1,2,2,3-tetrachloropropane, and 1,1,2,3-tetrachloropropane may be provided to a dehydrochlorination reactor 545, into which a dehydrochlorination reagent 550 is fed, in this case a caustic base, namely aqueous NaOH where a dehydrochlorination reaction is carried out. A phase transfer catalyst may be employed in the dehydrochlorination reactor 545 to facilitate dehydrochlorination of 1,2,2,3-tetrachloropropane, or 1,2,2,3-tetrachloropropane can be separated from the feed to dehydrochlorination reactor 545 and dehydrochlorinated in a separate dehydrochlorination reactor (not shown) with phase transfer catalyst.

In the dehydrochlorination reaction, the 1,2,3-trichloropropane converts to 2,3-dichloropropene, and the 1,1,2,3-tetrachloropropane and 1,2,2,3-tetrachloropropane convert to trichloropropenes. The dehydrochlorination reaction product 555 from the dehydrochlorination reactor 545 is provided to a separator 560, which may be a decanter, dryer, distillation column, or a combination of these, where water and NaCl byproduct are separated and removed in stream 570, and the purified product 565 is directed to the chlorination reactor 512. The purified product 565 includes 2,3-dichloropropene, 1,1,3-trichloropropene, 2,3,3-trichloropropene, and 1,2,3-trichloropropene, and may also contain unreacted tetrachloropropanes which may be fed to the first chlorination reactor 512, or while not shown in FIG. 2, may be separated out by a separator and recycled to the dehydrochlorination reactor 545 or another chlorination reactor (also not shown) which is a free radical chlorination reactor which employs free radical initiators such as AIBN.

Returning again to a second separator 540, leaving the second separator 540 is a heavy fraction 572 including 1,1,1,2,3-pentachloropropane, 1,1,2,2,3-pentachloropropane, and 1,1,2,3,3-pentachloropropane which is provided to a third separator 575, where a heavy stream 580 with 1,1,2,3,3-pentachloropropane is removed with other heavier compounds. A lighter purified product 585 having 1,1,1,2,3-pentachloropropane, 1,1,2,2,3-pentachloropropane are provided to a dehydrochlorination reactor 590 into which a dehydrochlorination reagent 595 is fed, in this case a caustic, namely NaOH where a dehydrochlorination reaction is carried out. The dehydrochlorination reaction product 600 is then provided to a separator 605, which may be a decanter, dryer, distillation column, or a combination of these, where water and NaCl byproduct are separated in stream 615 and removed.

A purified product 610 from the separator 605 including 1,1,2,3-tetrachloropropene, 2,3,3,3-tetrachloropropene and any unreacted pentachloropropanes, such as 1,1,1,2,3-pentachloropropane and 1,1,2,2,3-pentachloropropane, is provided to a separator 620 where product stream 625 is withdrawn including tetrachloropropenes such as 1,1,2,3-tetrachloropropene and 2,3,3,3-tetrachloropropene. Further, the heavies stream 627 with pentachloropropanes is provided back to separator 575 for separation of pentachloropropane components. In FIG. 2, although separators are shown for illustrative purposes, such processes may be omitted. Similarly, recycle streams may be omitted as well. While not shown in FIG 1 or FIG. 2, the product streams 265 or 625, respectively, may be fed to an isomerization reactor to produce 1,1,2,3-tetrachloropropene by processes known in the art.

In FIGS. 1 and 2, certain compounds are identified with shortened notation in the respective processes. The shorthand notation is such that each of the compounds are alkanes or alkenes having three carbons, the numbers indicate the positions of chlorine, and the use of "e" indicates -ene suffix, and the lack of "e" indicates -ane suffix.

### Notation:

- 3e:: 3-chloropropene
- PDC:: 1,2-dichloropropane
- 13e:: 1,3-dichloropropene
- 23e:: 2,3-dichloropropene
- 33e:: 3,3-dichloropropene
- 113e:: 1,1,3-trichloropropene
- 123e:: 1,2,3,-trichloropropene
- 123:: 1,2,3,-trichloropropane
- 233e:: 2,3,3,-trichloropropene
- 1123:: 1,1,2,3-tetrachloropropane
- 1223:: 1,2,2,3-tetrachloropropane
- 1123e:: 1,1,3,3-tetrachloropropene
- 2333e:: 2,3,3,3-tetrachloropropene
- 11123:: 1,1,1,2,3-pentachloropropane
- 11223:: 1,1,2,2,3-pentachloropropane
- 11233:: 1,1,2,3,3-pentachloropropane

As disclosed herein, the terms chloropropane and/or chloropropene optionally along with prefixes, the term di-, tri-, penta- encompass all isomers of the compound and all positions of the chlorine(s) along the hydrocarbon chain making up the propane or propene base chain. For instance, the term chloropropenes includes all isomers of chloropropene, including 1-chloropropene, 2-chloropropene, or 3-chloropropene. The term dichloropropenes includes all isomers of dichloropropene, including 1,3-dichloropropene, 2,3-dichloropropene, and 3,3-dichloropropene. The term dichloropropanes includes all isomers of dichloropropane including 1,2-dichloropropane. The term trichloropropenes includes all isomers of trichloropropene, including cis- and trans-, including for instance, 1, 1,3-trichloropropene, 2,3,3-trichloropropene, cis-1,2,3-trichloropropene, trans-1,2,3-trichloropropene. Similarly, the term trichloropropanes includes all isomers of trichloropropane, including 1,2,3-trichloropropane. The term tetrachloropropenes includes all isomers of tetrachloropropene including 1,1,2,3-tetrachloropropene and 2,3,3,3-tetrachloropropene. The term tetrachloropropanes includes all isomers of tetrachloropropane including 1,1,2,3-tetrachloropropane and 1,2,2,3-tetrachloropropane. The term pentachloropropanes includes all isomers of pentachloropropane including 1,1,1,2,3-pentachloropropane, 1,1,2,2,3-pentachloropropane, and 1,1,2,3,3-pentachloropropane.

### EXAMPLES

To facilitate understanding of the present disclosure, the following examples of certain embodiments are provided, and in no way should the following examples be read to limit the scope of the disclosure.

### Example 1: Preparation of 1,1,2,3-Tetrachloropropane

Into a 250 mL three necked round bottom flask equipped with a water condenser, magnetic stir bar, and an argon pad was charged with 100g of 1,3-dichloropropene (13e) (dried with molecular sieve AW 300). The 13e was stirred and heated to 55°C. Clz was bubbled at a rate of about 0.5 to 1g/min. The reaction mixture was sampled after 2 hours yielding 1,1,2,3-tetrachloropropane with greater than 90% selectivity.

### Example 2: Preparation of Trichloropropenes from 1,1,2,3-Tetrachloropropane

The product from Example 1 was distilled to produce a liquid mixture containing about 99.971 mole % 1,1,2,3-Tetrachloropropane, and 0.02 mole % 1,1,1,2,3-Pentachloropropane and 1,1,2,2,3- Pentachloropropane. A portion of this mixture weighing 50.5 g was added to a 250 ml round bottom flask. Stirring was begun and a purge of 10 sccm argon was started to the head space, the flask was heated to 95°C and 56.5 g of 20 weight % NaOH solution was dripped in over a period of 3 h. After addition of NaOH solution the reaction was kept at 94-95°C for an additional 2.5 h. At the end of the reaction, the product was cooled and the organic phase recovered. The organic phase was analyzed by GC, which indicated about 57.2 mole % 1,1,3-Trichloropropene 19.5 mole % 2,3,3-Trichloropropene, 7.2 mole % cis-1,2,3-Trichloropropene, 7.5 mole % trans-1,2,3-Trichloropropene. The conversion of 1,1,2,3-tetrachloropropane was 97 % with a selectivity of 94 % to useful trichloropropene isomers.

### Example 3: Preparation of Pentachloropropanes from Trichloropropenes

The trichloropropene mixture from Example 2 was combined with the product mixtures from other similar experiments and distilled to form a mixture containing about 89.1 mole % 1,1,3-Trichloropropene, 8.2 mole % trans-1,2,3-Trihloropropene and 2.5 mole % 2,3,3-Trichloropropene. 38.3 g of this material was transferred to a 100 ml round bottom flask equipped with a magnetic stirring bar and a condenser. The flask was heated to 50°C. 20 g chlorine was bubbled into the flask over a period of 2 h. The reaction was performed under a light argon stream. The flask temperature was maintained at 53°C. The product mixture was analyzed by GC, which indicated about 84.3 mole % 1,1,1,2,3-Pentachloropropane and 9.9 mole % 1,1,2,2,3-Pentachloropropane. The overall conversion of trichloropropenes was 97 % and the total selectivity to 1,1,2,2,3- and 1,1,1,2,3-Pentachloropropane was greater than 97 %.

### Example 4: Preparation of Tetrachloropropenes from Crude Pentachloropropanes

A portion of the crude pentachloropropane product mixture from Example 3 weighing 24.8 g was added to a 250 ml round bottom flask. Stirring was begun and a purge of 10 sccm argon was started to the head space. The flask was heated to 95°C and 27.2 g of 20 weight % NaOH solution was dripped in over a period of 3 h, after which stirring and temperature were maintained for an additional 2.5 h. At the end of the reaction, the product was cooled and the organic phase was recovered. The organic phase was analyzed by GC, which indicated about 42.0 mole % 1,1,2,3-Tetrachloropropene (1230xa) and 48.4 mole % 2,3,3,3-tetrachloropropene (1230xf). The conversion of 1,1,1,2,3-Pentachloropropane and 1,1,2,2,3-Pentachloropropane was >99.9 %. The selectivity of pentachloropropanes to tetrachloropropenes was about 90.1 %.

### Example 5: Preparation of 1,1,2,3-Tetrachloropropanefrom Crude 1,3-Dichloropropene

Into a 250 mL round bottom flask equipped with a cold water condenser, magnetic stir bar, and a heating mantle was charged 206.5 g of crude 1,3-dichloropropene comprising 22.2 mole % cis-1,3-dichloropropene, 18.3 mole % trans-1,3-dichloropropene, 51 mole % 1,2-dichloropropane, 3.4 mole % 2,3-dichloropropene, 2.0 mole % 3,3-dichloropropene and 1.4 mole % 3-chloropropene. The crude mixture was stirred and a mixture of 10 sccm nitrogen and 150 sccm chlorine gas was bubbled beneath the liquid surface. The mixture self-heated to a maximum temperature of 61°C. The average temperature over 4.3 h of chlorine feed was 48 °C. The final reaction mixture weighed 261.8 g and exhibited conversion of all olefinic species above 98 %. The mixture contained about 2.86 mole % 1,2,3-trichloropropane, 3.44 mole % 1,2,2,3-tetrachloropropane and 42.35 mole % 1,1,2,3-tetrachloropropane.

### Example 6: Preparation of Trichloropropenes from from Crude 1,2,2,3-Tetrachloropropane

The product from Example 5 was distilled to produce a liquid mixture containing about 2.66 mole % 1,2,3-trichloropropane, 4.62 mole % 1,2,2,3-tetrachloropropane, 89.70 mole % 1,1,2,3-tetrachloropropane and about 3 mole % impurities. A portion of this mixture weighing 111.3 g was added to a 500 ml round bottom flask with 20 g water. Stirring was begun and a purge of 10 sccm nitrogen was started to the head space. The flask was heated to 90°C and 128 g of 20 weight % NaOH was dripped in over a period of 3.9 hours. Maximum temperature during the reaction was 97°C. At the end of the reaction, the product was cooled and 78.6 g of the organic phase was recovered. The organic phase was analyzed by GC, which indicated about 51 mole % 1,1,3-trichloropropene and 31 mole % other trichloropropene isomers. The conversion of 1,1,2,3-tetrachloropropane was 98 % with a selectivity of 93 % to useful trichloropropene isomers.

### Example 7: Preparation of Pentachloropropanes from Crude Trichloropropenes

The crude trichloropropene mixture from Example 6 was combined with the product mixtures from other similar experiments to form a mixture containing about 49 mole % 1,1,3-trichloropropene and 31 mole % other trichloropropene isomers. 80.6 g of this material was transferred to a 100 ml round bottom flask without any purification or drying. Chlorine was bubbled into the flask for 3.1 hours with stirring at a rate of 135 sccm, along with nitrogen at 5 sccm. The flask temperature was maintained at 26°C - 30°C. The overall conversion of trichloropropenes was 97 % and the total selectivity to 1,1,2,2,3- and 1,1,1,2,3-pentachloropropanes was greater than 99 %.

### Example 8: Preparation of Tetrachloropropenes from Crude Pentachloropropanes

The crude pentachloropropane product mixture from Example 7 was distilled to remove most of the light components to obtain a mixture containing about 54 mole% 1,1,1,2,3-pentachloropropane, 35 mole% 1,1,2,2,3-pentachloropropane 1.7 mole% 1,2,2,3-tetrachloropropane 2.4 mole% 1,1,2,3-tetrachloropropane. A portion of this mixture weighing 91 g was added to a 500 ml round bottom flask with 20 g water. Stirring was begun and a purge of 10 sccm nitrogen was started to the head space, The flask was heated to 75°C and 79.5 g of 20 weight % NaOH was dripped in over a period of 2 hours, after which stirring and temperature were maintained for an additional 0.5 hours. At the end of the reaction, the product was cooled and 71.5 g of the organic phase was recovered. The organic phase was analyzed by GC, which indicated about 51.5 mole % 1,1,2,3-tetrachloropropene (1230xa) and 30.4 mole % 2,3,3,3-tetrachloropropene (1230xf). The conversion of 1,1,1,2,3-pentachloropropane was 93.3 % and 1,1,2,2,3-pentachloropropane was 96.7 %. The selectivity of pentachloropropanes to tetrachloropropenes was about 97.4 %. Most of the 1,1,2,3-tetrachloropropane in the starting material was converted to 1,1,3-trichloropropene.

## Claims

1. A process comprising:
reacting, in a first chlorination reaction, 1,3-dichloropropene with a chlorinating agent to form a first chlorination reaction product comprising a 1,1,2,3-tetrachloropropane;
reacting, in a first dehydrochlorination reaction, the first chlorination reaction product with a dehydrochlorination reagent or a dehydrochlorination catalyst to form a first dehydrochlorination reaction product comprising one or more trichloropropenes; and
reacting, in a second chlorination reaction, the first dehydrochlorination reaction product with the same or different chlorinating agent to form a second chlorination reaction product comprising at least one of 1,1,1,2,3-pentachloropropane or 1,1,2,2,3-pentachloropropane.

2. The process of claim 1, further comprising:
reacting, in a second dehydrochlorination reaction, the second chlorination reaction product with a dehydrochlorination reagent or a dehydrochlorination catalyst to form a second dehydrochlorination reaction product comprising at least one of 1,1,2,3-tetrachloropropene or 2,3,3,3-tetrachloropropene.

3. The process of claim 1 or 2, wherein the chlorinating agent is chlorine.

4. The process of any of claims 1-3, wherein the reaction of 1,3-dichloropropene and the chlorinating agent is in a liquid phase and the chlorinating agent is introduced as a gas or absorbed in a liquid solvent.

5. The process of any of claims 1-4, wherein the first dehydrochlorination reaction comprises reacting the first chlorination reaction product with the dehydrochlorination reagent.

6. The process of any of claims 1-5, wherein the dehydrochlorination reagent is an alkali metal hydroxide.

7. The process of claim 6, wherein the alkali metal hydroxide is NaOH.

8. The process of any of claims 1-7, wherein the dehydrochlorination catalyst in the first dehydrochlorination reaction comprises a Lewis acid catalyst.

9. The process of any of claims 1-8, wherein the conversion of 1,3-dichloropropene to 1,1,2,3-tetrachloropropane in the first chlorination reaction has at least 80% selectivity.

10. The process of any of claims 1-9, wherein the first chlorination reaction product is purified prior to the first dehydrochlorination reaction to obtain an increased concentration of 1,1,2,3-tetrachloropropane.

11. The process of any of claims 1-10, wherein the one or more trichloropropenes in the first dehydrochlorination reaction product are selected from the group of 1,1,3-trichloropropene, 2,3,3-trichloropropene, 1,2,3-trichloropropene, and mixtures thereof.

12. The process of any of claims 1-11, wherein the first dehydrochlorination reaction product is purified prior to the second chlorination reaction to obtain an increased concentration of the one or more trichloropropenes in the first dehydrochlorination reaction product.

13. The process of any of claims 1-12, wherein the chlorination of the one or more trichloropropenes in the second chlorination reaction produces a product that has at least 90% selectivity to 1,1,1,2,3-pentachloropropane, 1,1,2,2,3-pentachloropropane, or mixtures thereof.

14. The process of any of claims 1-13, wherein the 1,3-dichloropropene is provided as part of a crude dichloropropene stream, the crude dichloropropene stream further comprising at least one of 1,2-dichloropropane or isomers of dichloropropene other than 1,3-dichloropropene, the dichloropropene other than 1,3-dichloropropene comprising members selected from the group of 3,3-dichloropropene, 2,3-dichloropropene, and mixtures thereof, and
wherein reacting the 1,3-dichloropropene with the chlorinating agent in the first chlorination reaction comprises contacting the crude dichloropropene with the chlorinating agent to form the chlorination reaction product.

15. The process of claim 14, wherein the first chlorination reaction product further comprises 1,2,2,3-tetrachloropropane.

16. The process of claim 15, wherein at least a portion of the 1,2,2,3-tetrachloropropane is recycled to the first chlorination reaction.

17. The process of claim 15, wherein at least a portion of the 1,2,2,3-tetrachloropropane is fed to a separate chlorination reaction to which a free radical initiator is provided.

18. The process of claim 15, wherein at least a portion of the 1,2,2,3-tetrachloropropane is fed to a separate dehydrochlorination reaction to which a dehydrochlorination reagent and a phase transfer catalyst are provided.

19. The process of claim 15, further providing a phase transfer catalyst to the first dehydrochlorination reaction.

20. The process of claim 18 or 19, wherein the phase transfer catalyst comprises a quaternary ammonium salt.

21. The process of any of claim 1-20, wherein the first chlorination reaction and the second chlorination reaction are conducted together in a single chlorination reactor.

22. The process of claim 21, wherein the chlorination product from the single chlorination reactor is distilled to recover a stream enriched in tetrachloropropanes, which may be fed to the first dehydrochlorination reaction, and a stream comprising pentachloropropanes.

## Patentansprüche

1. Verfahren, umfassend:
Umsetzen von 1,3-Dichlorpropen mit einem Chlorierungsmittel in einer ersten Chlorierungsreaktion, um ein erstes Chlorierungsreaktionsprodukt zu bilden, das ein 1,1,2,3-Tetrachlorpropan umfasst;
Umsetzen des ersten Chlorierungsreaktionsprodukts in einer ersten Dehydrochlorierungsreaktion mit einem Dehydrochlorierungsreagens oder einem Dehydrochlorierungskatalysator, um ein erstes Dehydrochlorierungsreaktionsprodukt zu bilden, das ein oder mehrere Trichlorpropene umfasst; und
Umsetzen des ersten Dehydrochlorierungsreaktionsprodukts in einer zweiten Chlorierungsreaktion mit dem gleichen oder einem anderen Chlorierungsmittel, um ein zweites Chlorierungsreaktionsprodukt zu bilden, das mindestens eines von 1,1,1,2,3-Pentachlorpropan oder 1,1,2,2,3-Pentachlorpropan umfasst.

2. Verfahren nach Anspruch 1, ferner umfassend:
Umsetzen des zweiten Chlorierungsreaktionsprodukts in einer zweiten Dehydrochlorierungsreaktion mit einem Dehydrochlorierungsreagens oder einem Dehydrochlorierungskatalysator, um ein zweites Dehydrochlorierungsreaktionsprodukt zu bilden, das mindestens eines von 1,1,2,3-Tetrachlorpropen oder 2,3,3,3-Tetrachlorpropen umfasst.

3. Verfahren nach Anspruch 1 oder 2, wobei das Chlorierungsmittel Chlor ist.

4. Verfahren nach einem der Ansprüche 1-3, wobei die Reaktion von 1,3-Dichlorpropen und dem Chlorierungsmittel in einer flüssigen Phase erfolgt und das Chlorierungsmittel als ein Gas eingeführt oder in einem flüssigen Lösungsmittel absorbiert wird.

5. Verfahren nach einem der Ansprüche 1-4, wobei die erste Dehydrochlorierungsreaktion Umsetzen des ersten Chlorierungsreaktionsprodukts mit dem Dehydrochlorierungsreagens umfasst.

6. Verfahren nach einem der Ansprüche 1-5, wobei das Dehydrochlorierungsreagens ein Alkalimetallhydroxid ist.

7. Verfahren nach Anspruch 6, wobei das Alkalimetallhydroxid NaOH ist.

8. Verfahren nach einem der Ansprüche 1-7, wobei der Dehydrochlorierungskatalysator in der ersten Dehydrochlorierungsreaktion einen Lewis-Säure-Katalysator umfasst.

9. Verfahren nach einem der Ansprüche 1-8, wobei die Umwandlung von 1,3-Dichlorpropen in 1,1,2,3-Tetrachlorpropan in der ersten Chlorierungsreaktion eine Selektivität von mindestens 80 % aufweist.

10. Verfahren nach einem der Ansprüche 1-9, wobei das erste Chlorierungsreaktionsprodukt vor der ersten Dehydrochlorierungsreaktion gereinigt wird, um eine erhöhte Konzentration von 1,1,2,3-Tetrachlorpropan zu erhalten.

11. Verfahren nach einem der Ansprüche 1-10, wobei das eine oder die mehreren Trichlorpropene in dem ersten Dehydrochlorierungsreaktionsprodukt aus der Gruppe von 1,1,3-Trichlorpropen, 2,3,3-Trichlorpropen, 1,2,3- Trichlorpropen und Mischungen davon ausgewählt sind.

12. Verfahren nach einem der Ansprüche 1-11, wobei das erste Dehydrochlorierungsreaktionsprodukt vor der zweiten Chlorierungsreaktion gereinigt wird, um eine erhöhte Konzentration des einen oder der mehreren Trichlorpropene in dem ersten Dehydrochlorierungsreaktionsprodukt zu erhalten.

13. Verfahren nach einem der Ansprüche 1-12, wobei die Chlorierung des einen oder der mehreren Trichlorpropene in der zweiten Chlorierungsreaktion ein Produkt erzeugt, das eine Selektivität von mindestens 90 % für 1,1,1,2,3-Pentachlorpropan, 1,1,2,2,3-Pentachlorpropan oder Mischungen davon aufweist.

14. Verfahren nach einem der Ansprüche 1-13, wobei das 1,3-Dichlorpropen als Teil eines rohen Dichlorpropenstroms bereitgestellt wird, wobei der rohe Dichlorpropenstrom ferner mindestens eines von 1,2-Dichlorpropan oder Isomeren von Dichlorpropen, die von 1,3-Dichlorpropen verschieden sind, umfasst, wobei das von 1,3-Dichlorpropen verschiedene Dichlorpropen Elemente umfasst, die aus der Gruppe von 3,3-Dichlorpropen, 2,3-Dichlorpropen und Mischungen davon ausgewählt sind, und
wobei das Umsetzen des 1,3-Dichlorpropens mit dem Chlorierungsmittel in der ersten Chlorierungsreaktion Inkontaktbringen des rohen Dichlorpropens mit dem Chlorierungsmittel umfasst, um das Chlorierungsreaktionsprodukt zu bilden.

15. Verfahren nach Anspruch 14, wobei das erste Chlorierungsreaktionsprodukt ferner 1,2,2,3-Tetrachlorpropan umfasst.

16. Verfahren nach Anspruch 15, wobei mindestens ein Anteil des 1,2,2,3-Tetrachlorpropans in die erste Chlorierungsreaktion zurückgeführt wird.

17. Verfahren nach Anspruch 15, wobei mindestens ein Anteil des 1,2,2,3-Tetrachlorpropans einer separaten Chlorierungsreaktion zugeführt wird, für die ein freier Radikalinitiator bereitgestellt wird.

18. Verfahren nach Anspruch 15, wobei mindestens ein Anteil des 1,2,2,3-Tetrachlorpropans einer separaten Dehydrochlorierungsreaktion zugeführt wird, für die ein Dehydrochlorierungsreagens und ein Phasentransferkatalysator bereitgestellt werden.

19. Verfahren nach Anspruch 15, ferner einen Phasentransferkatalysator für die erste Dehydrochlorierungsreaktion bereitstellend.

20. Verfahren nach Anspruch 18 oder 19, wobei der Phasentransferkatalysator ein quaternäres Ammoniumsalz umfasst.

21. Verfahren nach einem der Ansprüche 1-20, wobei die erste Chlorierungsreaktion und die zweite Chlorierungsreaktion gemeinsam in einem einzigen Chlorierungsreaktor durchgeführt werden.

22. Verfahren nach Anspruch 21, wobei das Chlorierungsprodukt aus dem einzelnen Chlorierungsreaktor destilliert wird, um einen an Tetrachlorpropanen angereicherten Strom, der der ersten Dehydrochlorierungsreaktion zugeführt werden kann, und einen Pentachlorpropane umfassenden Strom zu gewinnen.

## Revendications

1. Procédé comprenant :
la réaction, dans une première réaction de chloration, de 1,3-dichloropropène avec un agent de chloration pour former un premier produit de réaction de chloration comprenant 1,1,2,3-tétrachloropropane ;
la réaction, dans une première réaction de déshydrochloration, du premier produit de réaction de chloration avec un réactif de déshydrochloration ou un catalyseur de déshydrochloration pour former un premier produit de réaction de déshydrochloration comprenant un ou plusieurs trichloropropènes ; et
la réaction, dans une deuxième réaction de chloration, du premier produit de réaction de déshydrochloration avec le même ou un autre agent de chloration pour former un deuxième produit de réaction de chloration comprenant au moins l'un de 1,1,1,2,3-pentachloropropane ou 1,1,2,2,3-pentachloropropane.

2. Procédé selon la revendication 1, comprenant en outre :
la réaction, dans une deuxième réaction de déshydrochloration, du deuxième produit de réaction de chloration avec un réactif de déshydrochloration ou un catalyseur de déshydrochloration pour former un deuxième produit de réaction de déshydrochloration comprenant au moins l'un de 1,1,2,3-tétrachloropropène ou 2,3,3,3-tétrachloropropène.

3. Procédé selon la revendication 1 ou 2, dans lequel l'agent de chloration est le chlore.

4. Procédé selon l'une quelconque des revendications 1-3, dans lequel la réaction du 1,3-dichloropropène et de l'agent de chloration est dans une phase liquide, et l'agent de chloration est introduit sous forme de gaz ou absorbé dans un solvant liquide.

5. Procédé selon l'une quelconque des revendications 1-4, dans lequel la première réaction de déshydrochloration comprend la réaction du premier produit de réaction de chloration avec le réactif de déshydrochloration.

6. Procédé selon l'une quelconque des revendications 1-5, dans lequel le réactif de déshydrochloration est un hydroxyde métallique alcalin.

7. Procédé selon la revendication 6, dans lequel l'hydroxyde métallique alcalin est NaOH.

8. Procédé selon l'une quelconque des revendications 1-7, dans lequel le catalyseur de déshydrochloration dans la première réaction de déshydrochloration comprend un catalyseur acide de Lewis.

9. Procédé selon l'une quelconque des revendications 1-8, dans lequel la conversion du 1,3-dichloropropène en 1,1,2,3-tétrachloropropane dans la première réaction de chloration a une s'électivité d'au moins 80 %.

10. Procédé selon les revendications 1-9, dans lequel le premier produit de réaction de chloration est purifié avant la première réaction de déshydrochloration pour obtenir une concentration accrue de 1,1,2,3-tétrachloropropane.

11. Procédé selon l'une quelconque des revendications 1-10, dans lequel le ou les trichloropropènes dans le premier produit de réaction de déshydrochloration sont sélectionnés dans le groupe constitué de 1,1,3-trichloropropène, 2,3,3-trichloropropène, 1,2,3-trichloropropène, et de mélanges de ceux-ci.

12. Procédé selon l'une quelconque des revendications 1-11, dans lequel le premier produit de réaction de déshydrochloration est purifié avant la deuxième réaction de chloration pour obtenir une concentration accrue du ou des trichloropropènes dans le premier produit de réaction de déshydrochloration.

13. Procédé selon l'une quelconque des revendications 1-12, dans lequel la chloration du ou des trichloropropènes dans la deuxième réaction de chloration produit un produit qui a une sélectivité d'au moins 90% au 1,1,1,2,3-pentachloropropane, au 1,1,2,2,3-pentachloropropane, ou à des mélanges de ceux-ci.

14. Procédé selon l'une quelconque des revendications 1-13, dans lequel le 1,3-dichloropropène est fourni comme faisant partie d'un courant de dichloropropène brut, le courant de dichloropropène brut comprenant en outre au moins l'un de 1,2-dichloropropane ou d'isomères de dichloropropène autre que 1,3-dichloropropène, le dichloropropène autre que 1,3-dichloropropène comprenant des éléments sélectionnés dans le groupe constitué de 3,3-dichloropropène, 2,3-dichloropropène, et de mélanges de ceux-ci, et
dans lequel la réaction du 1,3-dichloropropène avec l'agent de chloration dans la première réaction de chloration comprend la mise en contact du dichloropropène brut avec l'agent de chloration pour former le produit de réaction de chloration.

15. Procédé selon la revendication 14, dans lequel le premier produit de réaction de chloration comprend en outre 1,2,2,3-tétrachloropropane.

16. Procédé selon la revendication 15, dans lequel au moins une partie du 1,2,2,3-tétrachloropropane est recyclée à la première réaction de chloration.

17. Procédé selon la revendication 15, dans lequel au moins une partie du 1,2,2,3-tétrachloropropane est fournie à une réaction de chloration séparée à laquelle est fourni un initiateur de radicaux libres.

18. Procédé selon la revendication 15, dans lequel au moins une partie du 1,2,2,3-tétrachloropropane est fournie à une réaction de déshydrochloration séparée à laquelle sont fournis un réactif de déshydrochloration et un catalyseur de transfert de phase.

19. Procédé selon la revendication 15, fournissant en outre un catalyseur de transfert de phase à la première réaction de déshydrochloration.

20. Procédé selon la revendication 18 ou 19, dans lequel le catalyseur de transfert de phase comprend un sel d'ammonium quaternaire.

21. Procédé selon l'une quelconque des revendications 1-20, dans lequel la première réaction de chloration et la deuxième réaction de chloration sont exécutées ensemble dans un seul réacteur de chloration.

22. Procédé selon la revendication 21, dans lequel le produit de chloration provenant du seul réacteur de chloration est distillé pour récupérer un courant enrichi en tétrachloropropanes, qui puisse être fourni à la première réaction de déshydrochloration, et un courant comprenant des pentachloropropanes.
